# EUROPEAN PATENT SPECIFICATION

(11) **EP 2 060 567 B1**
(45) Date of publication and mention of the grant of the patent: **11.03.2015**
(21) Application number: 08253766.3
(22) Date of filing: 19.11.2008
(51) Int. Cl.: C07D 307/92

(54) **Regiospecific furan compounds and their use in fragrances**
Regiospezifische Furanverbindungen und ihre Anwendung als Duftstoffe
Composés régiospécifiques de furane et leur utilisation dans des parfums

(30) Priority: 19.11.2007 US 942020; 13.11.2008 US 270545
(43) Date of publication of application: 20.05.2009
(73) Proprietor: International Flavors & Fragrances, Inc., New York New York 10019 (US)
(72) Inventor: Boden, RIchard, Ocean, NJ 07712 (US); Jones, Paul Daniel, Matawan, NJ 07747 (US)
(74) Representative: Lawrence, John

(56) References cited:
- OPDYKE D L J ET AL: "Monographs on fragrance raw materials (part I)" FOOD AND CHEMICAL TOXICOLOGY 1982 GB, vol. 20, no. 6 Spec., 1982, page 695, XP002515194 ISSN: 0278-6915
- BERSUKER, I. B. ET AL: "Structural and electronic origin of ambergris odor of cyclic compounds" NOUVEAU JOURNAL DE CHIMIE , 9(3), 211-18 CODEN: NJCHD4; ISSN: 0398-9836, 1985, XP001537069

## Description

### Field of the Invention

The present invention relates to new chemical entities and the incorporation and use of the new chemical entities as fragrance materials.

### Background of the Invention

There is an ongoing need in the fragrance industry to provide new chemicals to give perfumers and other persons ability to create new fragrances for perfumes, colognes and personal care products. Those with skill in the art appreciate how differences in the chemical structure of the molecule can result in significant differences in the odor, notes and characteristics of a molecule. These variations and the ongoing need to discover and use the new chemicals in the development of new fragrances allow perfumers to apply the new compounds in creating new fragrances. One of the aims of a person skilled in the art of perfumery is to find new chemicals with improved performance from an olfactive point of view, either because their odor qualities are more distinctive and original or because their odor is much stronger or both of these. Such a skilled person knows well enough that they cannot rely on structural closeness to predict whether a new chemical will be a more interesting fragrance ingredient or, even, whether it will be fragrant at all.
The present invention is yet another example of this reality and it brings precisely a new and unexpected contribution into this field.

GRISALVA^{™} is described in Opdyke D.L.J. et al.: "Monographs on fragrance raw materials (part I)", Food and Chemical Toxicology, vol. 20, no. 6 Spec., 1982, page 695:

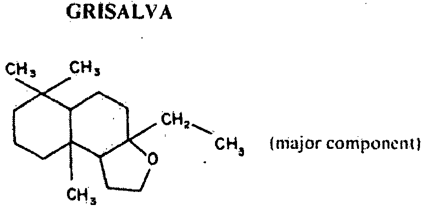

### Summary of the Invention

The present invention is concerned with diastereoisomer mixture of Structure I in accordance with claim 9 herein.

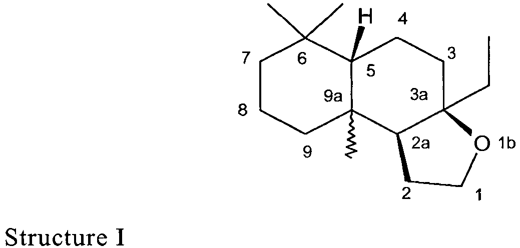

In accordance with the invention, a process in accordance with claim 1 herein is disclosed for synthesizing a diastereoisomer mixture of Structure I.

In a further embodiment of the invention Structure II 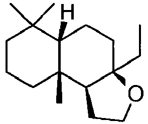 is provided in accordance with claim 4 herein.

In yet a further embodiment of the invention Structure III 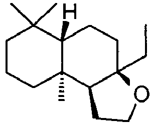 is provided in accordance with claim 5 herein.

According to another aspect of the invention, there is provided a process for the preparation of an isomeric component of structure II in accordance with claim 2 herein.

According to another aspect of the invention, there is provided a process for the preparation of an isomeric component of structure III in accordance with claim 3 herein.

According to another aspect of the invention, there is provided a method of improving, enhancing or modifying a fragrance formulation in accordance with claims 6, 7, 8, or 10 herein.

### Detailed Description of the Invention

It has been surprisingly discovered that the isomers and diastereoisomer mixture of the present invention possess superior organoleptic properties. In addition, the isomers and diastereoisomer mixture are distinctly advantageous in perfumery applications when compared to their racemic mixture counterparts known from the prior art.

In our improved process we have surprisingly found a regiospecific ring forming procedure which produces a diastereoisomer mixture and substantially pure individual isomeric components.

In the course of the present investigations it has been found that Structure I 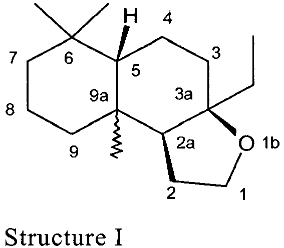 when compared to the commercially available GRISALVA^{™} possesses valuable odorant properties.

Structure I comprises the two isomers of Structure II and Structure III

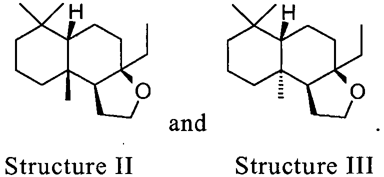

Structure I possesses more powerful, cleaner smelling, more ambery, woodier, ambergris fragrance notes. The buttery character in Structure I is also nicer, creamier and very appealing. There is a clear difference in the overall quality of Structure I, also apparent in the dry down. Even after eight hours on the blotter, Structure I appears to be stronger, woodier and more substantive.

It has been found that the compounds possess valuable odorant properties and can accordingly be used as odorants.

The olfactory notes of Structure I can be characterized as possessing nice woody, ambery, flowery, creamy, powerful, buttery, sweet notes. Structure II possesses woody, labdanum and ambergris notes and Structure III possesses woody, ambery ionone and strong fragrance notes.
On the basis of their olfactory notes, the diastereoisomer mixture of Structure I and the individual isomers, Structure II and III, are especially suitable for modifying and intensifying known compositions. In particular, their extraordinary olfactory strength, which contributes quite generally to the refinement of the compositions, should be emphasized.

One skilled in the art would recognize that manufactured fragrance compositions contain many different compounds at varying levels. For example, GRISALVA^{™} as commercially sold by International Flavors & Fragrances Inc. may contain at least fourteen different compounds. In Example VI, the Gas Chromatograph data of GRISALVA^{™} and the improved quality of the present invention is provided. The cis/trans ratio of Structure II and Structure III in GRISALVA^{™} is 14/5 as compared to the cis/trans ratio of Structure II and Structure III of about 41/34 obtained by the improved process. Accordingly, the diastereoisomer mixture may have a having a cis/trans isomer ratio of at least about 30/50 to about 50/30. The cis/trans ratio may also be in the range of about 35/45 to about 45/35. As understood by one skilled in the art the cis/trans isomer ratio can be increased by distillation and removing the front and back isomeric impurities.

A fragrance composition can contain a fragrance-enhancing amount of the Structure I wherein the diastereoisomer mixture may have a having a cis/trans isomer ratio of at least about 30/50 to about 50/30. The cis/trans ration may also be in the range of about 35/45 to about 45/35.

According to the present invention, the purity of Structure II and Structure III is considerably higher. Structure II has a purity level of at least about 70%, more preferably of at least about 80%, more preferably of about 90%, more preferably of about 95%, more preferably of about 97.5% and more preferably of about 99%. Structure III has a purity level of at least about 70%, more preferably of at least about 80%, more preferably of about 90%, more preferably of about 95%, more preferably of about 97.5% and more preferably of about 99%.

In accordance with the invention substantially pure is defined as a purity level of about 70% and greater.

A fragrance composition can contain a fragrance-enhancing amount of either Structure II or Structure III wherein each structure has a purity level of at least about 70%, more preferably of at least about 80%, more preferably of about 90%, more preferably of about 95%, more preferably of about 97.5% and more preferably of about 99%.

Furthermore a fragrance composition can contain both Structure II and Structure III wherein each structure has a purity level of at least about 70%, more preferably of at least about 80%, more preferably of about 90%, more preferably of about 95%, more preferably of about 97.5% and more preferably of about 99%.

Methods for improving, enhancing or modifying a fragrance formulation is also provided in accordance with claims 6, 7, 8, or 10 herein.

The use of the compounds of the present invention is widely applicable in current perfumery products, including the preparation of perfumes and colognes, the perfuming of personal care products such as soaps, shower gels, and hair care products as well as air fresheners and cosmetic preparations. The present invention can also be used to perfume cleaning agents, such as, but not limited to detergents, dishwashing materials, scrubbing compositions, window cleaners and the like.

In these preparations, the compounds of the present invention can be used alone or in combination with other perfuming compositions, solvents, adjuvants and the like. The nature and variety of the other ingredients that can also be employed are known to those with skill in the art.

Many types of fragrances can be employed in the present invention, the only limitation being the compatibility with the other components being employed. Suitable fragrances include but are not limited to fruits such as almond, apple, cherry, grape, pear, pineapple, orange, strawberry, raspberry; musk, flower scents such as lavender-like, rose-like, iris-like, carnation-like. Other pleasant scents include herbal and woodland scents derived from pine, spruce and other forest smells. Fragrances may also be derived from various oils, such as essential oils, or from plant materials such as peppermint, spearmint and the like.

A list of suitable fragrances is provided in US Pat. No. 4,534,891. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

Olfactory effective amount is understood to mean the amount of compound in perfume compositions the individual component will contribute to its particular olfactory characteristics, but the olfactory effect of the perfume composition will be the sum of the effects of each of the perfumes or fragrance ingredients. Thus the compounds of the invention can be used to alter the aroma characteristics of the perfume composition, or by modifying the olfactory reaction contributed by another ingredient in the composition. The amount will vary depending on many factors including other ingredients, their relative amounts and the effect that is desired.

The level of compound of the invention employed in the perfumed article varies from about 0.005 to about 10 weight percent, preferably from about 0.5 to about 8 and most preferably from about 1 to about 7 weight percent. In addition to the compounds other agents can be used in conjunction with the fragrance. Well known materials such as surfactants, emulsifiers, polymers to encapsulate the fragrance can also be employed without departing from the scope of the present invention.

Another method of reporting the level of the compounds of the invention in the perfumed composition, i.e., the compounds as a weight percentage of the materials added to impart the desired fragrance. The compounds of the invention can range widely from 0.005 to about 70 weight percent of the perfumed composition, preferably from about 0.1 to about 50 and most preferably from about 0.2 to about 25 weight percent. Those with skill in the art will be able to employ the desired level of the compounds of the invention to provide the desired fragrance and intensity.

Structure II and III are accessible according to the following Scheme:

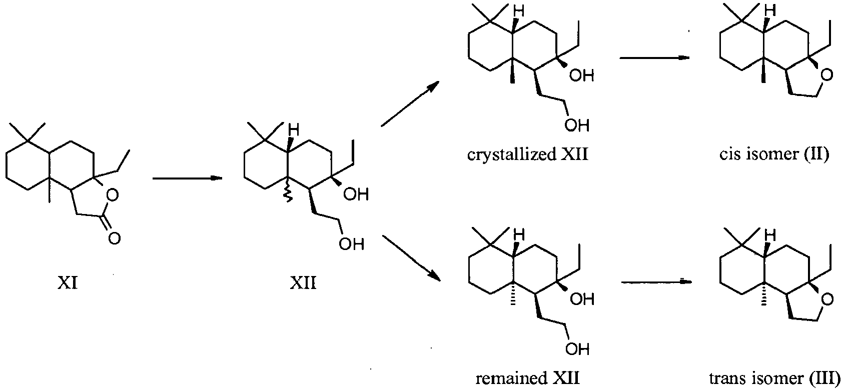

The individual steps involved in the process are based on chemistry known to one skilled in the art.

According to one of the embodiment of the invention, the preparation of an diastereoisomeric mixture of the structure, known by one skilled the art known by one skilled in the art as 3a-Ethyl-6,6,9a-trimethyl-dodecahydronapthol[2,1-b]furan: 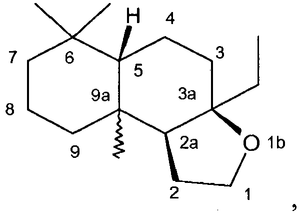 is prepared according to the following steps:
(1) Subjecting a compound of Structure IV, known by one skilled in the art as 1-(2,2,6-Trimethyl-cyclohexyl)-pentan-3-one, 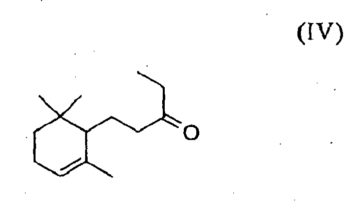 to Darzen's condensation to provide a compound of Structure V, 3-Ethyl-3-[2-(2,6,6-trimethyl-cyclohex-2-enyl)-ethyl]-oxirane-2-carboxylic acid methyl ester 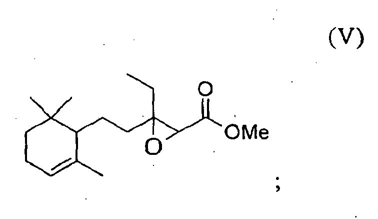
(2) saponifying the compound of Structure V to provide a compound of Structure VI, known by one skilled in the art as 3-ethyl-3-[2-(2,2,6-trimethyl-cyclohexyl)-ethyl]-oxirane-2-carboxylic acid, 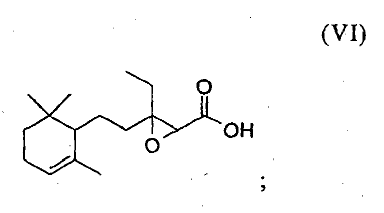
(3) subjecting the compound Structure VI to pyrolysis to provide a compound of Structure VII 2-ethyl-4-(2,6,6-trimethyl-cyclohex-2-enyl)-butyraldehyde] 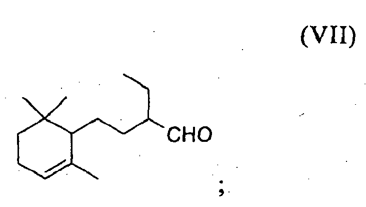
(4) subjecting a compound of structure VII to Knoevenagel reaction with cyanoacetic acid to provide a compound of structure VIII 4-ethyl-6-(2,6,6-trimethylcyclohex-2-enyl)hex-3-enenitrile 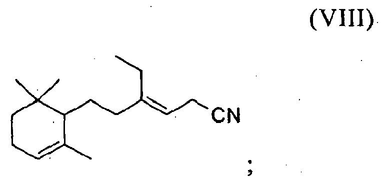
(5) providing a compound of structure IX, known by one skilled in the art as 2-ethyl-5,5,8a-trimethyl-3,4,4a,5,6,7,8,,8a-octahydro-naphthalen-1-yl)-acetonitrile by subjecting structure VIII to ring closing reaction 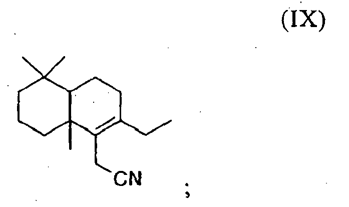
(6) saponifying the compound of structure IX to provide a compound of structure X, known by one skilled in the art as 2-ethyl-5,5,8a-trimethyl-3,4,4a,5,6,7,8,,8a-octahydro-naphthalen-1-yl)-acetic acid, 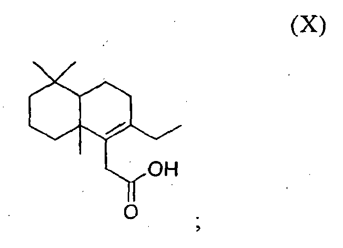
(7) cyclizing the compound of structure X to provide a compound of structure XI, known by one skilled in the art as 3a-Ethyl-6,6,9a-trimethyl-decahydro-naphthol[2,1-b]furan-2-one, 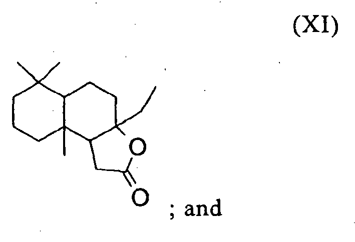
(8) reducing the compound of structure XI to provide the structure XII, known by one skilled in the art as 2-ethyl-1-(2-hydroxy-ethyl)-5,5,8a-trimethyl-decahydro-napthalen-2-ol, 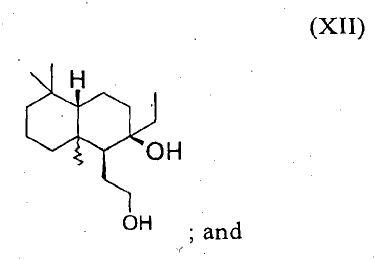
(9) subjecting structure XII to ring closing reaction to provide the diastereoisomeric mixture of structure I:

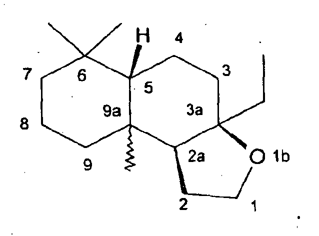

According to another embodiment of the invention, the individual isomeric components are obtained following the process detailed below:
(1) subjecting a compound of structure IV 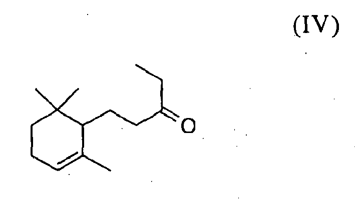 to Darzen's condensation to provide a compound of Structure V, 3-Ethyl-3-[2-(2,6,6-trimethyl-cyclohex-2-enyl)-ethyl]-oxirane-2-carboxylic acid methyl ester 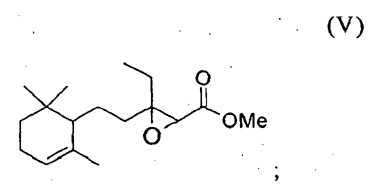
(2) saponifying the compound of Structure V to provide a compound of Structure VI, known by one skilled in the art as 3-ethyl-3-[2-(2,2,6-trimethyl-cyclohexyl)-ethyl]-oxirane-2-carboxylic acid, 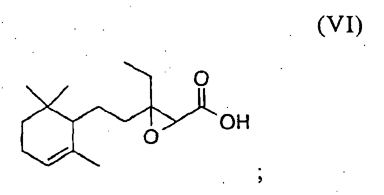
(3) subjecting the compound Structure VI to pyrolysis to provide a compound of Structure VII 2-ethyl-4-(2,6,6-trimethyl-cyclohex-2-enyl)-butyraldehyde 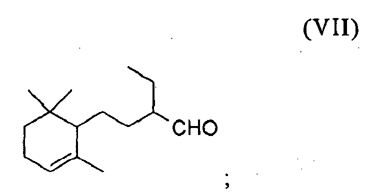
(4) subjecting a compound of structure VII to Knoevenagel reaction with cyanoacetic acid to provide a compound of structure VIII, 4-ethyl-6-(2,6,6-trimethylcyclohex-2-enyl)hex-3-enenitrile 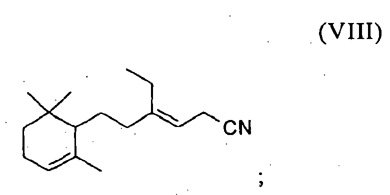
(5) providing a compound of structure IX, known by one skilled in the art as 2-ethyl-5,5,8a-trimethyl-3,4,4a,5,6,7,8,,8a-octahydro-naphthalen-1-yl)-acetonitrile by subjecting structure VIII to ring closing reaction 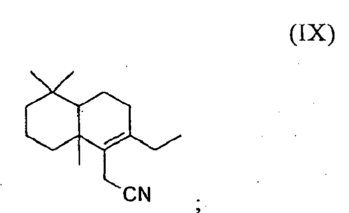
(6) saponifying the compound of structure IX to provide a compound of structure X 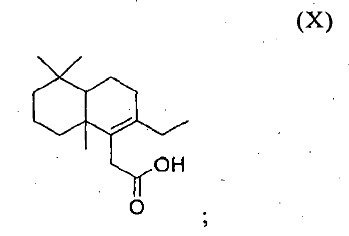
(7) cyclizing the compound of structure X to provide a compound of structure XI 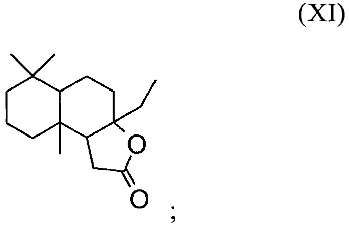 and
(8) reducing the compound of structure XI to provide structure XII 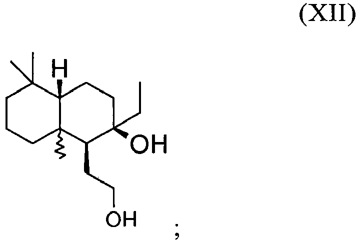 and
(9) crystallizing the compound of structure XII to form crystals and collecting the crystals via vacuum filtration and subjecting the crystals to a ring closure reaction and obtaining the cis isomer of Structure II,(3aR,5aS,9aR,9bS)-3a-ethyl-6,6,9a-trimethyldodecahydronaphtho[2,1-b]furan 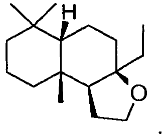 In an embodiment of the process to obtain the *trans* isomer of structure III, the following additional step may be performed.
(10) the remaining sample containing structure XII was heated, then cooled to room temperature and was seeded with the crystals collected in the earlier filtration. The crystals that formed were collected via vacuum filtration and the crystals were subjected to ring closure and the trans isomer of Structure III was obtained:
   (3aR,5aS,9aS,9bS)-3a-ethyl-6,6,9a-trimethyldodecahydronaphtho[2,1-b]furan

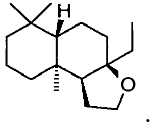

It is a matter of course that modifications concerning the reagents and reaction conditions are possible.

The following are provided as specific embodiments of the present invention. Other modifications of this invention within the scope of the claims will be readily apparent to those skilled in the art. Such modifications are understood to be within the scope of this invention. As used herein all percentages are weight percent unless otherwise noted, ppm is understood to stand for parts per million and g is understood to be grams. IFF as used in the examples is understood to mean International Flavors & Fragrances Inc., New York, NY, USA. All fragrance materials mentioned in the examples are available from IFF.

### Example I

### Preparation of the Diastereoisomeric Mixture and the Individual Isomeric Components

### Step 1: Darzen's condensation

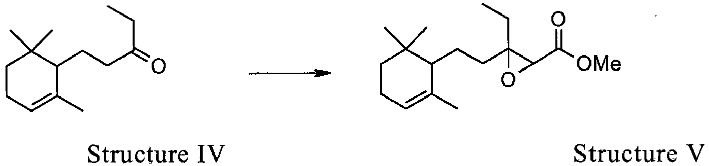

### Procedure:

To a 5 L flask under nitrogen was added toluene (1 L) and potassium *tert*-butoxide (336 g), and the stirred mixture was cooled to - 10 °C, Dihydromethyl ionone (582 g) was charged over 10 minutes while maintaining a temperature between -10 °C and 0 °C. Methyl chloroacetate (313 g) was charged over 4 hours such that the internal temperature did not exceed 0 °C.

The reaction stirred for an additional 1.5 hours while warming to room temperature (GC indicated an 80 % conversion) and the reaction was quenched by the addition of 2 L 3 % acetic acid solution. The layers were separated, and the organic layer was washed with water until neutral prior to purification via rushover distillation.

The crude ester was purified by distillation using a rushover apparatus consisting of a 3 L distillation flask equipped with a 2 inch (5,08 cm) splash column containing stainless steel mesh, a rushover column, and a fraction cutter. The material was distilled with 25 g of primol.

### Step 2: Saponification of glycic ester

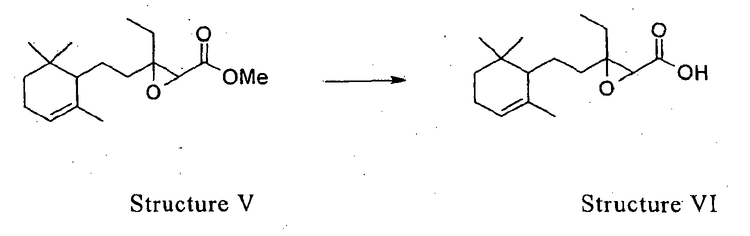

### Procedure:

The ester (1014 g) and methanol (2 L) was charged under nitrogen to a 5 L flask, and the reaction was cooled to 0 - 5 °C. Sodium hydroxide was fed over 15 minutes (exothermic) such that the reaction temperature did not exceed 20 °C. The reaction was stirred at room temperature for 2 hours and was poured into water (2 L) and toluene (800 mL) - the mixture was stirred and the organic layer was discarded. The aqueous layer was extracted with toluene (800 mL) - discard organic layer. The aqueous layer was returned to the reaction flask and was cooled to 5 °C. Phosphoric acid was fed into the stirred solution (exothermic) over 15 minutes such that the reaction temperature did not exceed 20 °C. The crude reaction mixture was poured into a separatory funnel and the layers were separated.

### Step 3: Pyrolysis of carboxylic acid

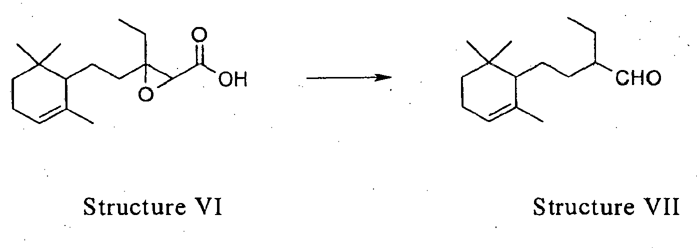

### Procedure:

Primol was added to the 3L flask and the system was placed under vacuum (5 - 9 mm Hg, i.e. 667-1200 Pa).

After heating the primol to 210 °C, the crude acid mixture was fed into the primol over 6.5 hours - product distills during feed. After the feed was complete, the vacuum was increased to 1.5 mm Hg (200 Pa) and held for 15 minutes. The distilled mixture was washed with saturated sodium bicarbonate, then brine and was purified by rushover distillation.

The crude aldehyde was purified by distillation using a rushover apparatus consisting of a 2 L distillation flask equipped with a 2" (50,8 mm) splash column containing stainless steel mesh, a rushover column, and a fraction cutter.

### Step 4: Knoevenagel reaction

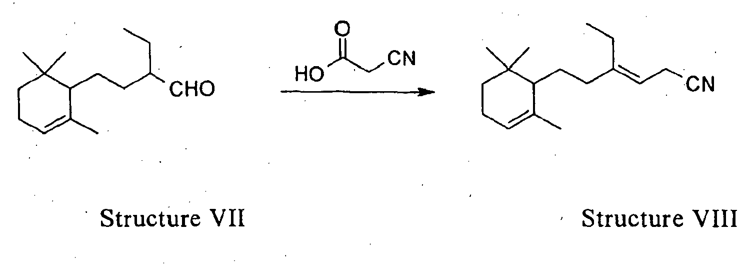

### Procedure:

The aldehyde was charged, under nitrogen, to the 3 L reaction flask followed by cyanoacetic acid, DMF, 2-ethylhexylamine, and acetic acid. With stirring, the mixture was placed under vacuum (120 mm; 16 KPa) and was heated to 80 - 85 °C. Note: Cyanoacetic acid begins to decompose near 90 °C. During the reaction, water and some DMF distilled from reaction. When the distillation ended (35 minutes), the vacuum was increased to 70 mm, and the reaction stirred for an additional 30 minutes (additional water distilled from the reaction) - check for remaining starting material (GC). Note: Do not raise temperature above 85 °C, as cyanoacetic acid will begin to decompose near 90 °C. Additional cyanoacetic acid, 2-ethylhexylamine, and acetic acid (0.1 eq each) may be added. The reaction was removed from vacuum, the rushover apparatus was removed, a 12inch (30,5 cm) reflux column connected to a gas bubbler was attached, and acetic anhydride was added. The reaction was heated to 130 °C, and a vigorous evolution of CO₂ was observed. After CO₂ evolution stopped (2 h), the temperature was raised to 140 °C and the reaction stirred for an additional 30 minutes or until additional CO₂ evolution stopped. The reaction was cooled to 50 °C, poured into 500 mL water, was stirred and the layers were separated (pH aq = 5-6).

The aqueous layer was extracted with saturated sodium bicarbonate (200 mL), and the crude product was purified by fractional distillation.

The crude nitrile was purified by fractional distillation using a 24 inch (61 cm) goodloe-packed column. The material was distilled with 101 g of primol.

### Step 5: Phosphoric Acid catalyzed ring closure

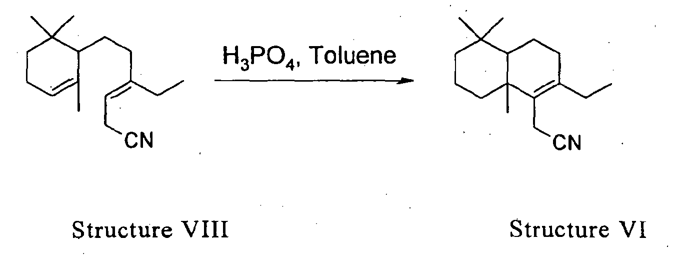

### Procedure:

The nitrile was charged under nitrogen to a 2 liter flask followed by toluene and phosphoric acid. The reaction was heated to reflux and stirred for 2 hours, after which time, 450 mL toluene was removed via bidwell. The reaction was cooled to 50 °C, poured into water (1 L) and toluene (250 mL), and was stirred and the layers were separated. The aqueous layer was extracted with toluene (250 mL) and the combined organics were washed with saturated sodium bicarbonate, then brine. Solvent was removed via Rot-o-vap (80 °C, 40mm = 5,33 KPa), and the crude bicyclic nitrile was used in the next step without further purification.

### Step 6: Saponification of nitrile to acid

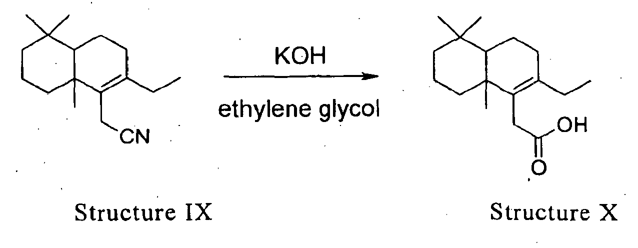

### Procedure:

The bicyclic nitrile was charged under nitrogen to a 3 L flask followed by ethylene glycol and potassium hydroxide, and was heated to reflux for 4.5 hours.

Reflux began at 150 °C, however as water vapor is released through the condenser, the reaction temperature increased to approx. 170 °C. The reaction was cooled, poured into water (1.5 L) and toluene (750 mL), was stirred, the layers were separated and the organic layer was discarded. The aqueous layer was extracted with toluene (250 mL x 2) - discard organics. The aqueous layer was acidified with hydrochloric acid (conc.) to a pH of 1, and was extracted with toluene (750 mL, then 250 mL). The combined organics were dried via azeotropic distillation using a 3 L 3-neck flask fitted equipped with a mechanical stirrer, heating mantle, thermocouple, a 12" (30,5 cm) reflux condenser, and a bidwell. The crude solution of product in toluene was used without further purification in the next step.

### Step 7: Amberlyst-15 catalyzed cyclization of acid (BC ring)

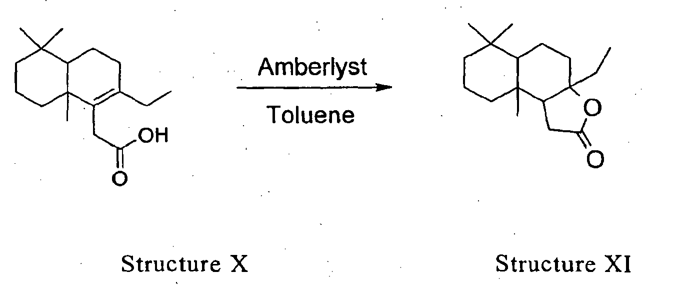

### Procedure:

The toluene solution of the bicyclic acid was charged under nitrogen to a 3 L flask, Amberlyst-15 was added, the reaction was heated to reflux for 10 hours. The reaction was cooled and filtered, and the Amberlyst resin was washed with toluene (250 ml). Solvent was removed via Rot-o-vap (80 °C, 40mm = 5,33 KPa), and the crude GRISALVA lactone was purified by rushover distillation.

The crude lactone was purified by distillation using a rushover apparatus consisting of a 500 mL distillation flask equipped with a 2inch (50,8 mm) splash column containing stainless steel mesh, a rushover column, and a fraction cutter, no cooling water was added to the condensor.

### Reduction of lactone to diol

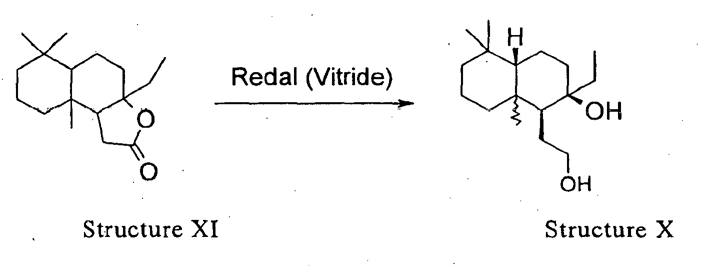

Toluene (1794 g) and lactone were charged under nitrogen to a 5 L flask, and the resulting solution was heated to 80 °C. Vitride was fed over 1 hour (exothermic) while maintaining 80 °C, and the reaction stirred for an additional 2 hours. The reaction temperature was cooled to 75 °C, and ethyl acetate was added (mild exotherm). The reaction stirred at 75 °C for 30 minutes, and a preheated 75 °C solution of 2.5 % sodium hydroxide (1 L) was added. The reaction was stirred for 1 hour and the layers were separated. The organic layer was washed with hot water (500 mL x 3) until the pH of the water wash was 7.5. The crude product in toluene was dried via azeotropic distillation using a 5 L 3-neck flask equipped with a mechanical stirrer, heating mantle, thermocouple, a 12 inch (30,5 cm) reflux condenser, and a bidwell. The crude reaction product was subjected to ring closure and the resulting mixture was confirmed by GC.

The diastereomeric mixture possesses nice woody, amber and flowery notes.

### Isolation of the Isomeric Components

Approximately 100 mL solvent removed during this procedure. The material cooled to room temperature and stood for 12 hours (overnight). The crystals that had formed were collected via vacuum filtration to give 110 g (after drying) rigid, colorless needles that were sparingly soluble in toluene. The crystals were subjected to ring closure and the resulting ether had the same GC retention time as cis isomer of GRISALVA. The stereochemistry of the diol collected and the ether formed were therefore assigned as: 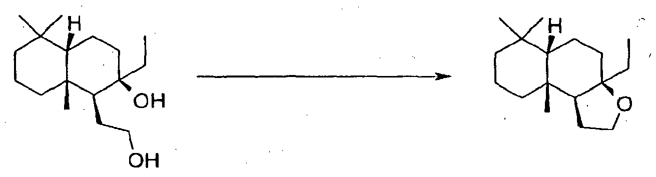 The cis isomer of Structure II possesses woody, labdanum and ambergris notes.

The cis isomer of Structure II was found to have a purity of 95.4%. The analytical data was obtained using a 6890N Gas Chromatograph commercially available from Agilent Technologies.

HMNR 0.82 ppm(s, 3H);0.88 ppm(t, 3H);0.89 ppm(s, 3H);1.10 ppm(s, 3H);1.23 ppm(m, 1H);1.25 ppm(m, 1H);1.32 ppm(m, 1H);1.32 ppm(m, 1H);1.40 ppm(m, 1H);1.42 ppm(m, 1H);1.43 ppm(m, 1H);1.43 ppm(m, 1H);1.53 ppm(m, 1H);1.60 ppm(m, 1H);1.65 ppm(m, 1H);1.67 ppm(m, 1H);1.67 ppm(m, 1H);1.72 ppm(m, 1H);1.83 ppm(m, 1H);2.03 ppm(m, 1H);3.62 ppm(m, 1H);3.80 ppm(m, 1H)

The mother liquor was heated to reflux and 1.2 L toluene was removed. The resulting solution cooled to room temperature, was seeded with the crystals collected in the earlier filtration, and stood for 12 hours (overnight). The crystals that formed were collected via vacuum filtration to give 60 g (after drying) of fine white crystals that were readily soluble in toluene.

The crystals were subjected to ring closure and the resulting ether had the same GC retention time as trans isomer of GRISALVA. The stereochemistry of the diol collected and the ether formed were therefore tentatively assigned as: 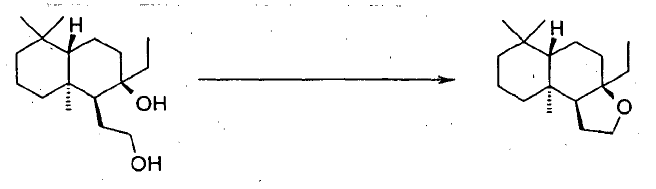 The trans isomer of Structure III possesses a woody, ambery ionone and strong fragrance notes.

The trans isomer of Structure III was found to have a purity of 87.3%. The analytical data was obtained using a 6890N Gas Chromatograph commercially available from Agilent Technologies.

HMNR 0.80 ppm(s, 3H);0.89 ppm(t, 3H, J = 6.5 Hz);1.02 ppm(bd, 1H, J = 13.5 Hz);1.07 ppm(s, 3H);1.11 ppm(s, 3H);1.14 ppm(d, 1H, J = 13.5 Hz, of t, J = 4.4 Hz);1.21 ppm(d, 1H, J = 12 Hz, of d, J = 4 Hz);1.33 ppm(d, 1H, J = 13 Hz, of t, J = 4.5 Hz);1.37 ppm(d, 1H, J = 13 Hz, of q, J = 4 Hz);1.42 ppm(m, 1H);1.49 ppm(m, 1H);1.57 ppm(d, 1H, J = 14 Hz, of t, J = 4 Hz);1.61 ppm(d, 1H, J = 14 Hz, of q, J = 6.5 Hz);1.63 ppm(m, 1H);1.66 ppm(bt, 1H, J = 9 Hz);1.67 ppm(m, 1H);1.71 ppm(d, 1H, J = 14 Hz, of q, J = 6.5 Hz);1.74 ppm(d, 1H, J = 14 Hz, of t, J = 4 Hz);1.97 ppm(m, 1H);2.07 ppm(m, 1H);3.67 ppm(m, 1H);3.82 ppm(m, 1H);

### Example II

### Incorporation of 3a-Ethyl-6,6,9a-trimethyl-dodecahydro-napthol[2,1-b]furan into a fragrance formulation

| | |
|---|---|
| Ambrettolide | 40 |
| Bicyclononalactone | 23 |
| Cashmeran® | 15 |
| Damascone Delta | 1 |
| Dihydro Myrcenol | 50 |
| Ethylene Brassylate | 140 |
| Geranium Oil African | 6 |
| 3a-Ethyl-6,6,9a-trimethyl-dodecahydro -napthol[2,1-b]furan | 50 |
| Iso E Super® | 325 |
| Kohinool® | 50 |
| Lemon Oil | 55 |
| Linalool | 15 |
| Linalyl Acetate | 25 |
| Mandarin Oil Md LMR | 45 |
| Methyl Dihydro Jasmonate | 100 |
| Orange Oil | 20 |
| Sanjinol | 40 |
| TOTAL | 1000 |

This is a musky, citrus floral accord designed for alcoholic fragrances.

### Example III

### Incorporation of (3aR,5aS,9aR,9bS)-3a-ethyl-6,6,9a-trimethyldodecahydronaphtho[2,1-b]furan into a fragrance formulation

| | |
|---|---|
| Triplal® | 5 |
| Benzyl Salicylate | 55 |
| Bourgeonal | 30 |
| Citronellol | 15 |
| Cyclamal | 10 |
| Dihydro Myrcenol | 75 |
| Dimethyl Benzyl Carbinyl Acetate | 35 |
| Fleuramone | 8 |
| Floralozone | 4 |
| (3ar,5as,9ar,9bs)-3a-Ethyl-6,6,9a-Trimethyldodecahydronaphtho[2,1-B]Furan | 10 |
| Cis-3-Hexenyl Salicylate | 15 |
| Hexyl Cinnamic Aldehyde | 200 |
| Hexyl Salicylate | 60 |
| Lilial | 200 |
| Methyl Dihydro Jasmonate | 85 |
| Muskalactone | 30 |
| Orange Oil | 20 |
| Tetrahydro Myrcenol | 75 |
| Undecanal | 3 |
| Zenolide | 65 |
| Total | 1000 |

This is a floral, balsamic, ambery accord designed for alcoholic fragrances.

### Example IV

### Incorporation of (3aR,5aS,9aS,9bS)-3a-ethyl-6,6,9a-trimethyldodecahydronaphtho[2,1-b]furan into a fragrance formulation

| Ingredient | Parts |
|---|---|
| (3ar,5as,9as,9bs)-3a-Ethyl-6,6,9a-Trimethyldodecahydronaphtho | |
| [2,1-B]Furan | 1 |
| Bornafix .Rtm | 3 |
| Citranalva | 1 |
| Cedrafix | 2.5 |
| Celestolide | 4 |
| Citrus Oil Distilled | 12 |
| Cyclacet | 3 |
| Dihydro Myrcenol | 40 |
| Fleuranil | 1 |
| Geranium Bourbon Oliffac | 0.5 |
| Hexyl Cinnamic Aldehyde | 4.5 |
| Iso E Super | 2.5 |
| Kharismal | 4 |
| Koavone | 1.5 |
| Linalyl Acetate | 5 |
| Phenoxanol | 5 |
| Precyclemone B | 1.5 |
| Pseudo Linalyl Acetate | 5 |
| Styralyl Acetate | 1 |
| Vigoflor | 1 |
| Zenolide | 1 |
| Total | 100 |

This is a citrus fragrance.

### Example V

The following is a comparative example between the GRISALVA^{™} composition commercially available from IFF Inc. and Structure I. The odor evaluations were performed by a trained GC perfumer and a panelist of perfumers.

The fragrance notes are as follows:
**GRISALVA^{™} Composition:** woody, ambergris, slightly heavy, not as buttery as Structure I, ambery.

**Structure I:** c reamy, woodier, powerful, ambery, buttery, sweeter, good body.

The odor quality of Structure I is more powerful, cleaner smelling, more ambery, woodier, ambergris that the commercially available GRISALVA^{™}. The buttery character in the new material is nicer, creamier, very appealing. There is a clear difference in the overall quality, also apparent in the dry down. After eight hours on blotter, Structure I appears to be stronger, woodier, more substantive.
In Table I the fragrance descriptions of strength, substantivity and quality are assessed on a scale from 1 - 10, with 1 being the low end of the scale and 10 being the high end of the scale. Dry down are determined after 6 hours.

**Table I**

| **GRISALVA^{™}** | **Product @ 0.1% in Alcohol "Fresh"** | **Product @ 0.1% in Alcohol "Dry Down"** |
|---|---|---|
| Strength | 5.0 | 3.0 |
| Substantivity | 5.0 | 2.0 |
| Quality | 5.0 | 3.0 |
| | | |

| **Structure I** | | |
|---|---|---|
| Strength | 5.5 | 4.0 |
| Substantivity | 6.0 | 4.5 |
| Quality | 7.0 | 4.5 |

**Table II**

| **GRISALVA^{™}** | **Product @ 1.0% in Alcohol "Fresh"** | **Product @ 1.0% in Alcohol "Dry Down"** |
|---|---|---|
| Strength | 5.5 | 3.5 |
| Substantivity | 6.0 | 4.0 |
| Quality | 5.5 | 4.5 |
| | | |

| **Structure I** | | |
|---|---|---|
| Strength | 6.5 | 4.0 |
| Substantivity | 7.5 | 5.5 |
| Quality | 8.0 | 6.0 |

**Table III**

| **GRISALVA^{™}** | **Product @ 10.0% in Alcohol "Fresh"** | **Product @ 10.0% in Alcohol "Dry Down"** |
|---|---|---|
| Strength | 7.0 | 6.0 |
| Substantivity | 7.0 | 6.0 |
| Quality | 7.0 | 6.5 |
| | | |

| **Structure I** | | |
|---|---|---|
| Strength | 9.0 | 7.0 |
| Substantivity | 8.0 | 7.5 |
| Quality | 8.5 | 8.0 |

### EXAMPLE VI

The following example provides analytical data which demonstrates a significant difference in the composition between the two products. The analytical data was obtained using a 6890N Gas Chromatograph shows.
Peak 4 and Peak 5 correlate to Structure III and Structure II.

| **Current Quality of GRISALVA^{™}** | **Percent (%)** |
|---|---|
| Peak 1 | 1.17 |
| Peak 2 | 1.90 |
| Peak 3 | 8.80 |
| **Peak 4** | **5.14** |
| **Peak 5** | **13.98** |
| Peak 6 | 1.60 |
| Peak 7 | 7.60 |
| Peak 8 | 1.10 |
| Peak 9 | 8.52 |
| Peak 10 | 5.63 |
| Peak 11 | 2.30 |
| Peak 12 | 12.45 |
| Peak 13 | 15.74 |
| Peak 14 | 1.85 |
| Peak 15 | 7.84 |

Remaining difference completed by minor peaks totaling 100%.

| **Improved Quality,** | **Percent (%)** |
|---|---|
| Peak 1 | 4.28 |
| Peak 2 | 3.10 |
| Peak 3 | 4.59 |
| **Peak 4** | **33.82** |
| **Peak 5** | **41.23** |
| Peak 6 | 1.31 |
| Peak 7 | 1.07 |

Remaining difference completed by minor peaks totaling 100.0%.

Compared to the commercial mixture the improved quality contains much higher levels of both Peak 4 and Peak 5. The cis/trans ratio of the diastereoisomer is about 41/33 in the improved quality as compared to about 14/5 in the commercial grade.

### Example VII

To further demonstrate the organoleptic properties of each variant, two formulas were prepared dosing each one equally. Dilutions were then prepared at a level of 10.0% in ethanol and evaluated. The following fragrance contains the commercially available GRISALVA^{™}:

| **Description:** | **Parts:** |
|---|---|
| ALD C-8 + BHT | 0.10 |
| Anise Star Oil USP | 2.00 |
| Bay Oil For CMPD | 0.10 |
| Benzoin Resinoid Special BLO | 0.40 |
| Cashmeran | 0.10 |
| Citral Extra Nat | 0.10 |
| Citronellol Coeur | 5.00 |
| Civet Artif Ind/Arom XBS W/O MX | 0.10 |
| Coumarin | 1.80 |
| Dimeth Benz Carb Acet | 0.50 |
| Dipropylene Glycol | 20.00 |
| Galaxolide 50 PCT Dep | 1.00 |
| Geranium African | 0.20 |
| GRISALVA | 5.20 |
| Hexenol, B, Gamma Extra | 0.70 |
| Kharismal | 11.30 |
| Lavandin Abrialis Special | 0.50 |
| Lemon Oil Cal CP"PFG" | 5.00 |
| Linalool Syn | 3.00 |
| Linalyl Acet | 8.00 |
| Lyral BHT | 6.00 |
| Meth Anth (USDEA) | 0.20 |
| Musk Z 4 | 1.00 |
| Patchouli Oil Light BLO | 10.00 |
| Santaliff BHT | 2.00 |
| Spearmint Oil | 2.50 |
| Stabiliff | 0.10 |
| Tangerine Oil FLA "PFG" | 2.00 |
| Terpinolene Rect BHT | 0.50 |
| Vanillin Ex Lighnin | 0.60 |
| Vertofix Coeur | 8.00 |
| Ylang 1 | 2.00 |
| **Total:** | 100.00 |

The fragrance formula is described as possessing lavendaceous, minty, sweet, citrusy, vanillic, herbaceous and woody notes.

### Example VIII

To further demonstrate the organoleptic properties of each variant, two formulas were prepared dosing each one equally. Dilutions were then prepared at a level of 10.0% in ethanol and evaluated. The following fragrance contains Structure I:

| **Description:** | **Parts:** |
|---|---|
| Ald C-8 + BHT | 0.10 |
| Anise Star Oil USP | 2.00 |
| Bay Oil For CMPD | 0.10 |
| Benzoin Resinoid Special Blo | 0.40 |
| Cashmeran | 0.10 |
| Citral Extra Nat | 0.10 |
| Citronellol Coeur | 5.00 |
| Civet Artif Ind/ Arom XBS W/O MX | 0.10 |
| Coumarin | 1.80 |
| Dimeth Benz Carb Acet | 0.50 |
| Dipropylene Glycol | 20.00 |
| Structure I: | 5.20 |
| Galaxolide 50 PCT Dep | 1.00 |
| Geranium African | 0.20 |
| Hexenol, B, Gamma Extra | 0.70 |
| Kharismal | 11.30 |
| Lavandin Abrialis Special | 0.50 |
| Lemon Oil Cal CP"PRG" | 5.00 |
| Linalool Syn | 3.00 |
| Linalyl Acet | 8.00 |
| Lyral BHT | 6.00 |
| Meth Anth (USDEA) | 0.20 |
| Musk Z 4 | 1.00 |
| Patchouli Oil Light BLO | 10.00 |
| Santaliff BHT | 2.00 |
| Spearmint Oil | 2.50 |
| Stabiliff | 0.10 |
| Tangerine Oil FLA"PFG" | 2.00 |
| Terpinolene Rect BHT | 0.50 |
| Vanillin Ex Lignin | 0.60 |
| Vertofix Coeur | 8.00 |
| Ylang 1 | 2.00 |
| | |
| **Total:** | 100.00 |

The fragrance containing structure I is a much fresher finished product, accentuating the sweet minty, woody and ambery top notes, also more lavendaceous and herbaceous. Formula containing Structure I is also perceived as being stronger, cleaner and crispier. The addition of Structure I allows for fragrance enhancement, increasing the overall woodiness and citrus notes giving the impression of more dimensions and a fuller body.

## Claims

1. A process for the preparation of an diastereoisomeric mixture of the following formula: 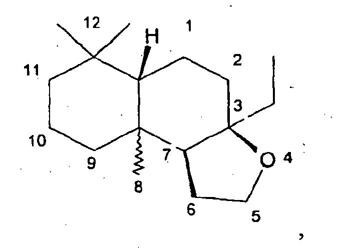 comprising the steps of:
(1) subjecting a compound of structure IV 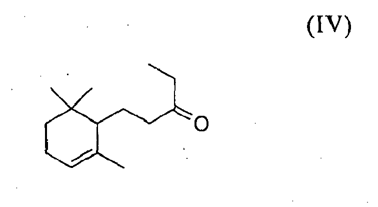 to Darzen's condensation to provide a compound of structure V 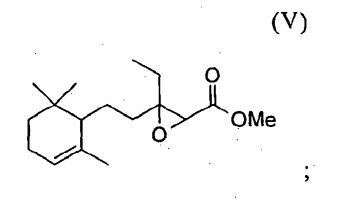
(2) saponifying the compound of structure V to provide a compound of structure VI 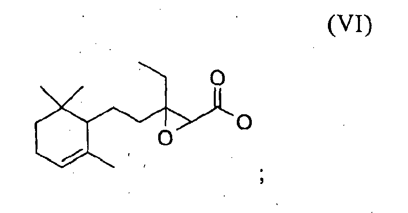
(3) subjecting the compound of structure VI to pyrolysis to provide a compound of structure VII 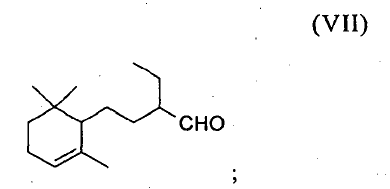
(4) subjecting a compound of structure VII to Knoevenagel reaction with cyanoacetic acid to provide a compound of structure VIII 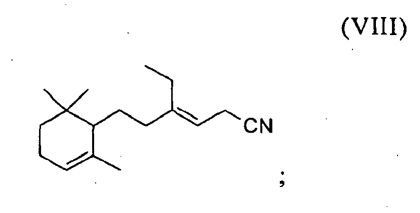
(5) providing a compound of structure IX by subjecting compound VIII to ring closing reaction 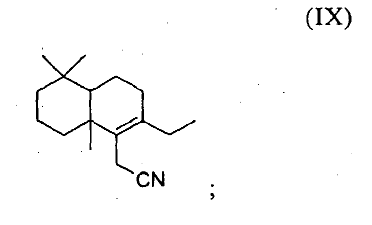
(6) saponifying the compound of structure IX to provide a compound of structure X 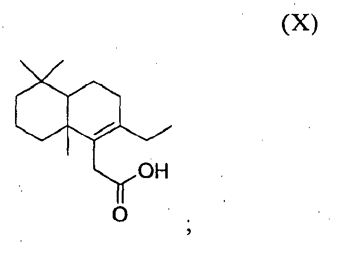
(7) cyclizing the compound of structure X to provide a compound of structure XI 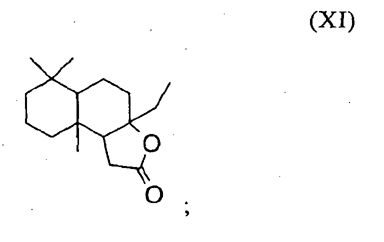 and
(8) reducing the compound of structure XI to provide a structure XII 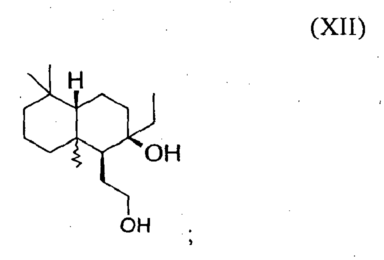 and
(9) subjecting a diastereoisomeric mixture of the structure XII to ring closing reaction to provide the diastereoisomeric mixture of structure I:

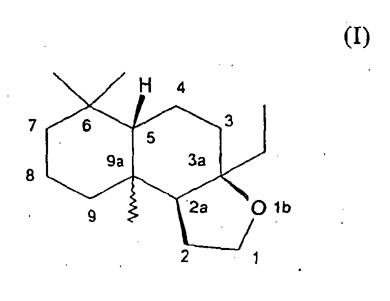

2. A process for the preparation of an isomeric component of the formula 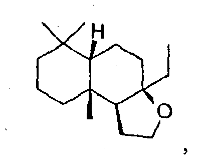 comprising the steps of:
(1) subjecting a compound of structure IV 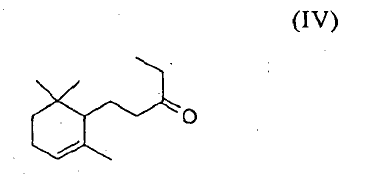 to Darzen's condensation to provide a compound of structure V 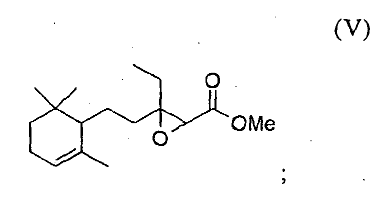
(2) saponifying the compound of structure V to provide a compound of structure VI 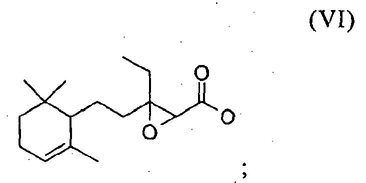
(3) subjecting the structure VI to pyrolysis to provide a compound of structure VII 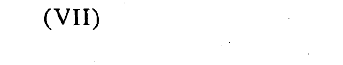 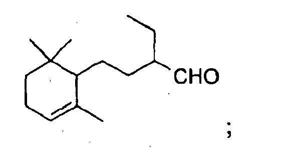
(4) subjecting a compound of structure VII to Knoevenagel reaction with cyanoacetic acid to provide a compound of structure VIII 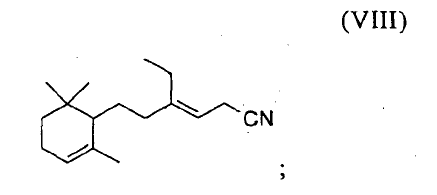
(5) providing a compound of structure IX by subjecting compound VIII to ring closing reaction . 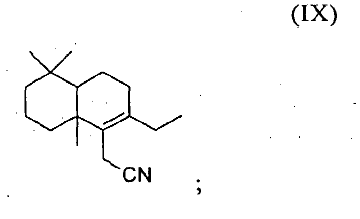
(6) saponifying the compound of structure IX to provide a compound of structure X 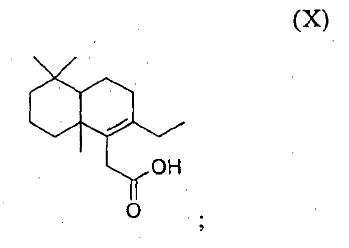
(7) cyclizing the compound of structure X to provide a compound of structure XI 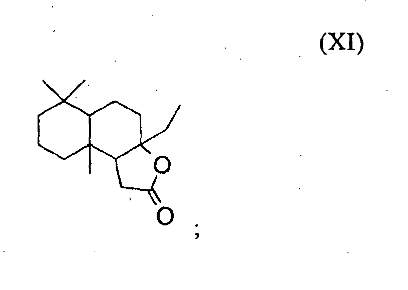 and
(8) reducing the compound of structure XI to provide structure XII 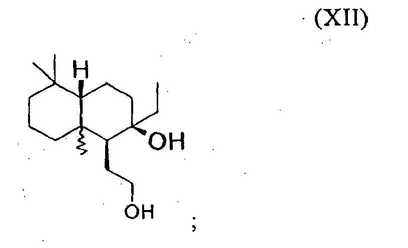 and
(9) crystallizing the compound of structure XII to form crystals and subjecting the crystals to a ring closure reaction and obtaining the cis isomer of Structure II:

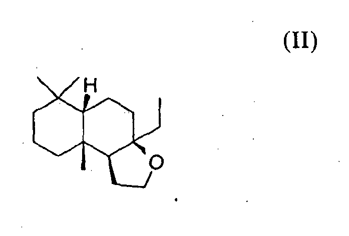

3. A process for the preparation of an isomeric component of the formula 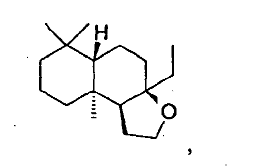 comprising the steps of:
(1) subjecting a compound of structure IV 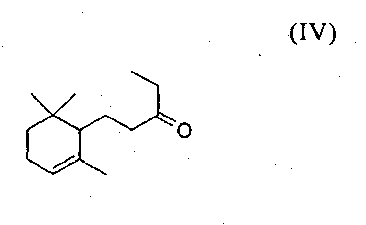 to Darzen's condensation to provide a compound of structure V 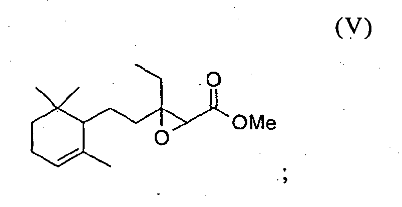
(2) saponifying the compound of structure V to provide a compound of structure VI 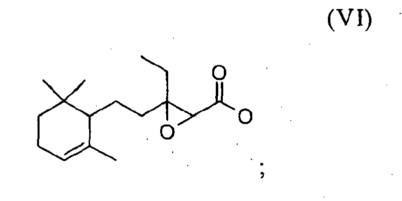
(3) subjecting the compound of structure VI to pyrolysis to provide a compound of structure VII 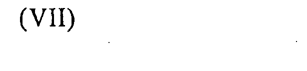 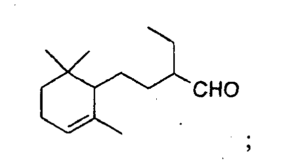
(4) subjecting a compound of structure VII to Knoevenagel reaction with cyanoacetic acid to provide a compound of structure VIII 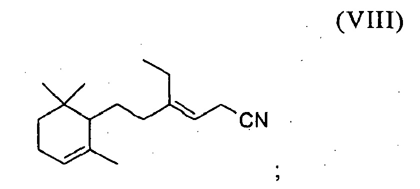
(5) providing a compound of structure IX by subjecting compound VIII to ring closing reaction 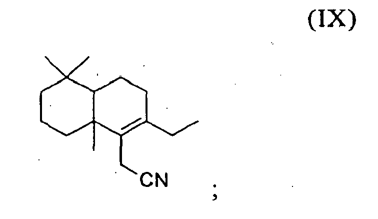
(6) saponifying the compound of structure IX to provide a compound of structure X 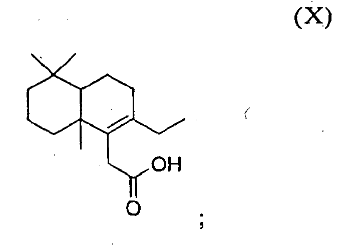
(7) cyclizing the compound of structure X to provide a compound of structure XI 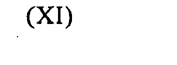 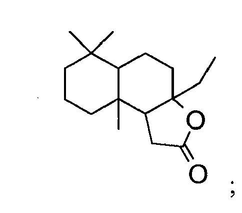 and
(8) reducing the compound of structure XI to provide structure XII 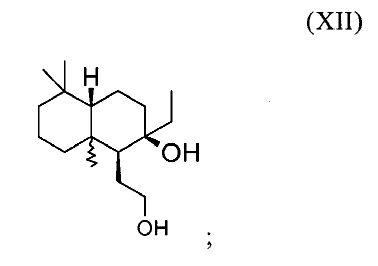 and
(9) crystallizing the compound of structure XII to form crystals and subjecting the crystals to a ring closure reaction and obtaining the cis isomer of Structure II: 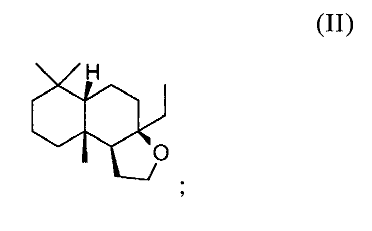 and
(10) crystallizing the remaining sample containing structure XII to form crystals and subjecting the crystals to a ring closure reaction and obtaining the trans isomer of Structure III:

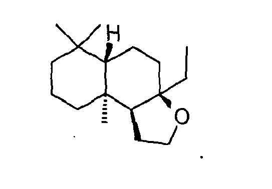

4. A compound: 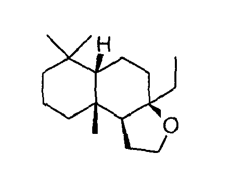 having a purity of at least:-
(i) 70%; or
(ii) 80%; or
(iii) 90%; or
(iv) 95%; or
(v) 97.5%; or
(vi) 99%.

5. A compound: 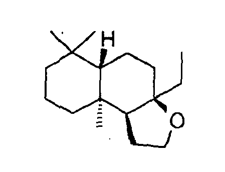 having a purity of at least:-
(i) 70%; or
(ii) 80%; or
(iii) 90%; or
(iv) 95%; or
(v) 97.5%; or
(vi) 99%.

6. A method of improving, enhancing or modifying a fragrance formulation through the addition of an olfactory acceptable amount of a compound: 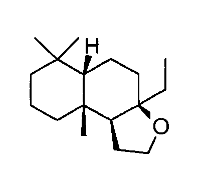 wherein the compound has a purity of at least:-
(i) 70%; or
(ii) 80%; or
(iii) 90%; or
(iv) 95%; or
(v) 97.5%; or
(vi) 99%.

7. A method of improving, enhancing or modifying a fragrance formulation through the addition of an olfactory acceptable amount of a compound: 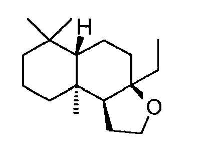 wherein the compound has a purity of at least:-
(i) 70%; or
(ii) 80%; or
(iii) 90%; or
(iv) 95%; or
(v) 97.5%; or
(vi) 99%.

8. A method of improving, enhancing or modifying a fragrance formulation through the addition of an olfactory acceptable amount of the following compounds: 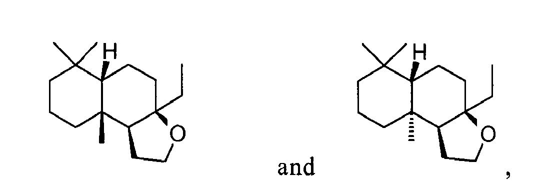 wherein each compound has a purity of at least:-
(i) 70%; or
(ii) 80%; or
(iii) 90%; or
(iv) 95%; or
(v) 97.5%; or
(vi) 99%.

9. A diastereoisomer mixture of the structure: 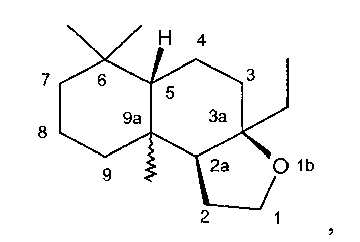 wherein the diastereoisomer mixture has a cis/trans isomer ratio of:-
(i) 30/50 to 50/30; or
(ii) 35/45 to 45/35; or
(iii) 41/34; or
(iv) 30/45.

10. A method of improving, enhancing or modifying a fragrance formulation through the addition of an olfactory acceptable amount of a diastereoisomer mixture of the structure: 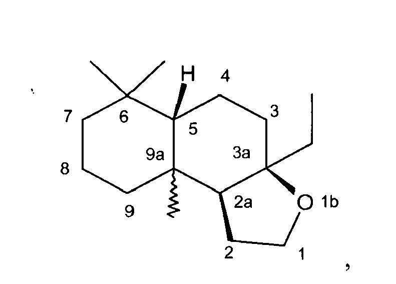 wherein the diastereoisomer mixture has a cis/trans isomer ratio of:-
(i) 30/50 to 50/30; or
(ii) 35/45 to 45/35; or
(iii) 41/34; or
(iv) 30/45.

## Patentansprüche

1. Ein Verfahren zur Vorbereitung einer diastereoisomerischen Mischung mit der folgenden Rezeptur: 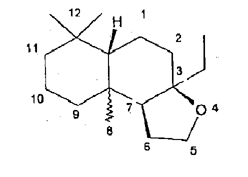 bestehend aus den Schritten:
(1) Durchführung an einer Verbindung der Struktur IV 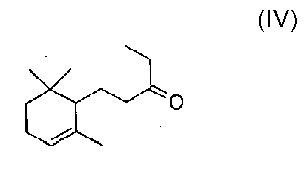 einer Darzens-Glycidester-Kondensation, um eine Verbindung der Struktur V zu erhalten 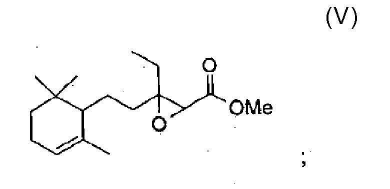
(2) Verseifung der Verbindung der Struktur V, um eine Verbindung der Struktur VI zu erhalten 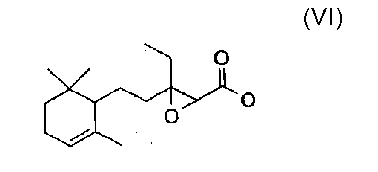
(3) Durchführung einer Pyrolyse an der Verbindung der Struktur VI, um eine Verbindung der Struktur VII zu erhalten 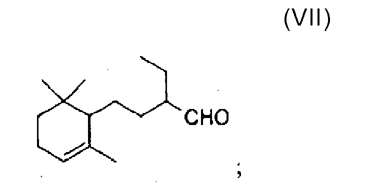
(4) Durchführung einer Knoevenagel-Reaktion mit Cyanessigsäure an einer Verbindung der Struktur VII, um eine Verbindung der Struktur VIII zu erhalten 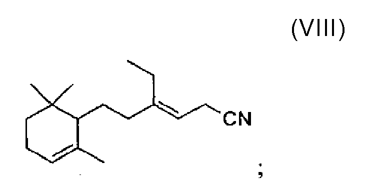
(5) Bereitstellung einer Verbindung der Struktur IX anhand der Durchführung einer Ringschlussreaktion an der Verbindung VIII 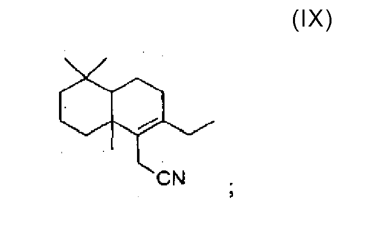
(6) Verseifung der Verbindung der Struktur IX, um eine Verbindung der Struktur X zu erhalten 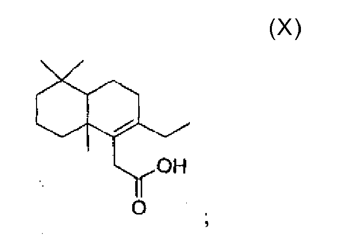
(7) Cyclisierung der Verbindung der Struktur X, um eine Verbindung der Struktur XI zu erhalten 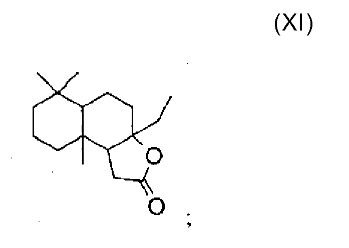 und
(8) Reduzierung der Verbindung der Struktur XI, um eine Verbindung der Struktur XII zu erhalten 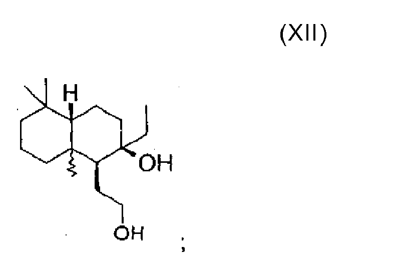 und
(9) Durchführung einer Ringschlussreaktion an einer diastereoisomerischen Mischung der Struktur XII, um die diastereoisomerische Mischung der Struktur I zu erhalten:

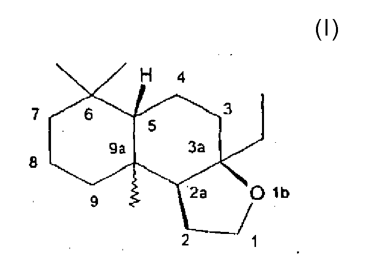

2. Ein Verfahren zur Vorbereitung einer isomerischen Komponente der Rezeptur, 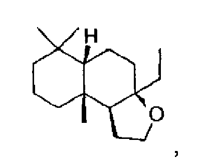 bestehend aus den Schritten:
(1) Durchführung an einer Verbindung der Struktur IV 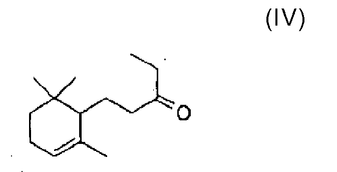 einer Darzens-Glycidester-Kondensation, um eine Verbindung der Struktur V zu erhalten 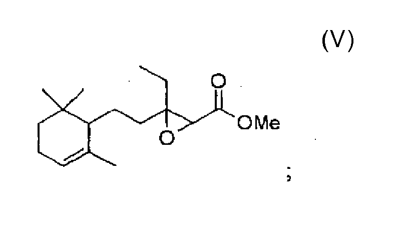
(2) Verseifung der Verbindung der Struktur V, um eine Verbindung der Struktur VI zu erhalten 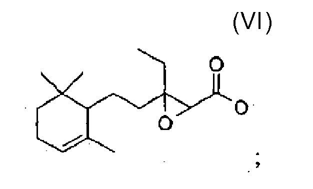
(3) Durchführung einer Pyrolyse an der Verbindung der Struktur VI, um eine Verbindung der Struktur VII zu erhalten 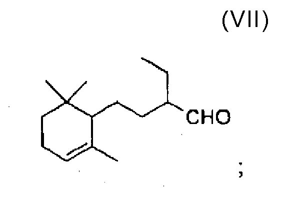
(4) Durchführung einer Knoevenagel-Reaktion mit Cyanessigsäure an einer Verbindung der Struktur VII, um eine Verbindung der Struktur VIII zu erhalten 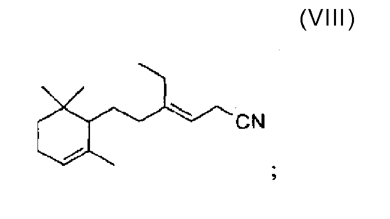
(5) Bereitstellung einer Verbindung der Struktur IX anhand der Durchführung einer Ringschlussreaktion an der Verbindung VIII 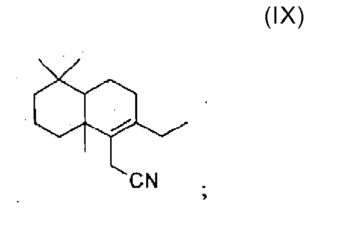
(6) Verseifung der Verbindung der Struktur IX, um eine Verbindung der Struktur X zu erhalten 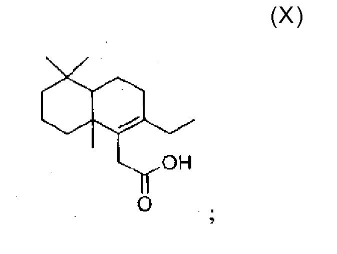
(7) Cyclisierung der Verbindung der Struktur X, um eine Verbindung der Struktur XI zu erhalten 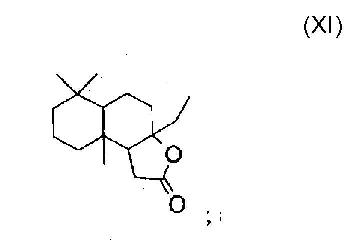 und
(8) Reduzierung der Verbindung der Struktur XI, um eine Verbindung der Struktur XII zu erhalten 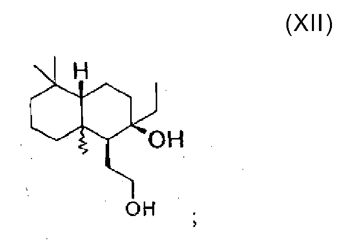 und
(9) Kristallisierung der Verbindung der Struktur XII zur Bildung von Kristallen und Durchführung einer Ringschlussreaktion an den Kristallen und Erlangung des cis-Isomers der Struktur II:

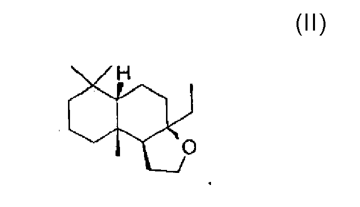

3. Ein Verfahren zur Vorbereitung einer isomerischen Komponente der Rezeptur, 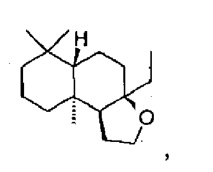 bestehend aus den Schritten:
(1) Durchführung an einer Verbindung der Struktur IV 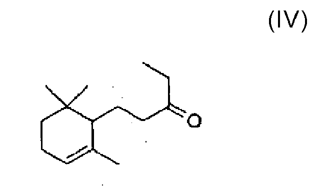 einer Darzens-Glycidester-Kondensation, um eine Verbindung der Struktur V zu erhalten 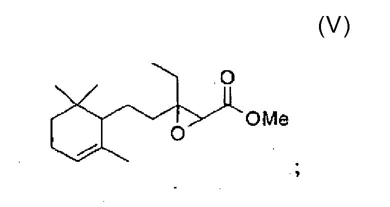
(2) Verseifung der Verbindung der Struktur V, um eine Verbindung der Struktur VI zu erhalten 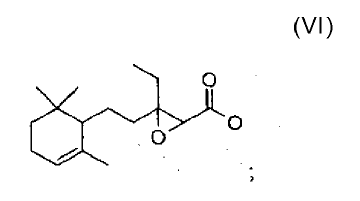
(3) Durchführung einer Pyrolyse an der Verbindung der Struktur VI, um eine Verbindung der Struktur VII zu erhalten 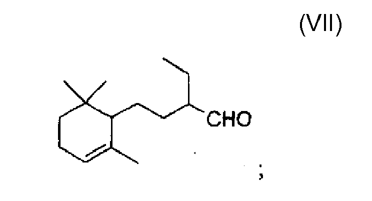
(4) Durchführung einer Knoevenagel-Reaktion mit Cyanessigsäure an einer Verbindung der Struktur VII, um eine Verbindung der Struktur VIII zu erhalten 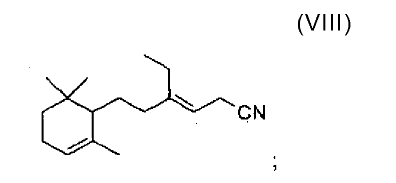
(5) Bereitstellung einer Verbindung der Struktur IX anhand der Durchführung einer Ringschlussreaktion an der Verbindung VIII 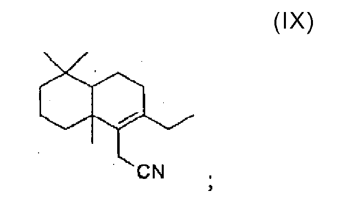
(6) Verseifung der Verbindung der Struktur IX, um eine Verbindung der Struktur X zu erhalten 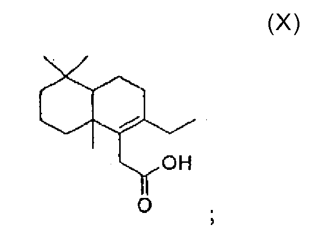
(7) Cyclisierung der Verbindung der Struktur X, um eine Verbindung der Struktur XI zu erhalten 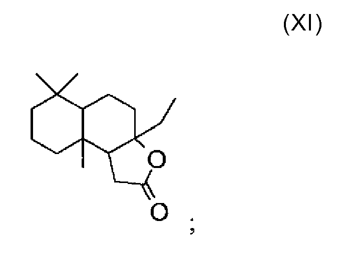 und
(8) Reduzierung der Verbindung der Struktur XI, um eine Verbindung der Struktur XII zu erhalten 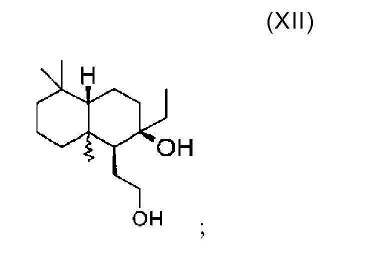
(9) Kristallisierung der Verbindung der Struktur XII zur Bildung von Kristallen und Durchführung einer Ringschlussreaktion an den Kristallen und Erlangung des cis-Isomers der Struktur II: 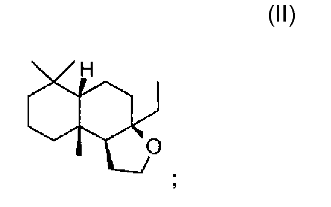 und
(10) Kristallisierung der verbleibenden Probe, die die Struktur XII enthält, um Kristalle zu bilden, und Durchführung einer Ringschlussreaktion an den Kristallen und Erlangung des trans-Isomers der Struktur III:

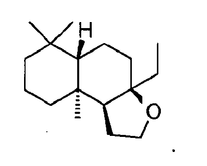

4. Eine Verbindung: 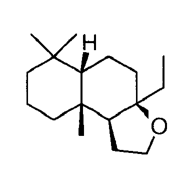 mit einer Reinheit von mindestens:-
(i) 70%; oder
(ii) 80%; oder
(iii) 90%; oder
(iv) 95%; oder
(v) 97,5%; oder
(vi) 99%.

5. Eine Verbindung: 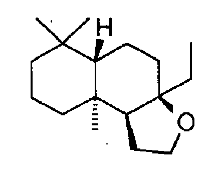 mit einer Reinheit von mindestens:-
(i) 70%; oder
(ii) 80%; oder
(iii) 90%; oder
(iv) 95%; oder
(v) 97,5%; oder
(vi) 99%.

6. Eine Methode zur Verbesserung, Verstärkung oder Veränderung einer Duftrezeptur durch Hinzufügung einer olfaktorisch akzeptablen Menge einer Verbindung: 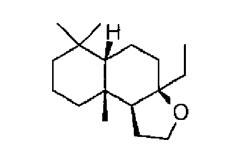 wobei die Verbindung eine Reinheit aufweist von mindestens:-
(i) 70%; oder
(ii) 80%; oder
(iii) 90%; oder
(iv) 95%; oder
(v) 97,5%; oder
(vi) 99%.

7. Eine Methode zur Verbesserung, Verstärkung oder Veränderung einer Duftrezeptur durch Hinzufügung einer olfaktorisch akzeptablen Menge einer Verbindung: 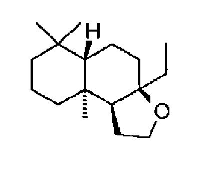 wobei die Verbindung eine Reinheit aufweist von mindestens:-
(i) 70%; oder
(ii) 80%; oder
(iii) 90%; oder
(iv) 95%; oder
(v) 97,5%; oder
(vi) 99%.

8. Eine Methode zur Verbesserung, Verstärkung oder Veränderung einer Duftrezeptur durch Hinzufügung einer olfaktorisch akzeptablen Menge der folgenden Verbindungen: 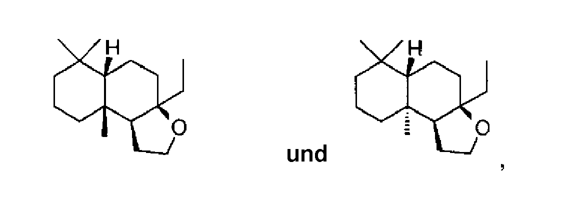 wobei jede Verbindung eine Reinheit aufweist von mindestens:-
(i) 70%; oder
(ii) 80%; oder
(iii) 90%; oder
(iv) 95%; oder
(v) 97,5%; oder
(vi) 99%.

9. Eine diastereoisomere Mischung der Struktur: 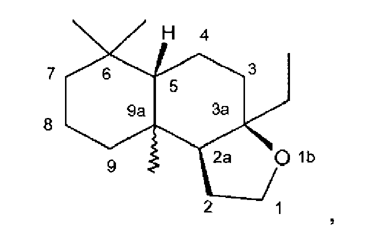 wobei die diastereoisomere Mischung ein cis/trans-Isomer-Verhältnis aufweist von:-
(i) 30/50 bis 50/30; oder
(ii) 35/45 bis 45/35; oder
(iii) 41/34; oder
(iv) 30/45.

10. Eine Methode zur Verbesserung, Verstärkung oder Veränderung einer Duftrezeptur durch Hinzufügung einer olfaktorisch akzeptablen Menge einer diastereoisomeren Mischung der Struktur: 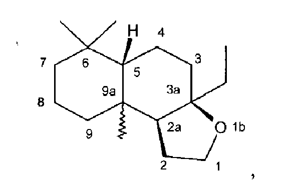 wobei die diastereoisomere Mischung ein cis/trans-Isomer-Verhältnis aufweist von:-
(i) 30/50 bis 50/30; oder
(ii) 35/45 bis 45/35; oder
(iii) 41/34; oder
(iv) 30/45.

## Revendications

1. Un processus de préparation d'un mélange diastéréo-isomérique de la formule suivante : 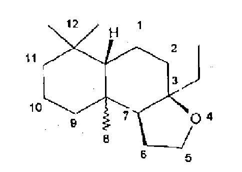 comprenant les opérations suivantes :
(1) la soumission d'un composé de la structure IV 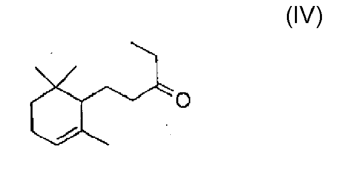 à une condensation de Darzens de façon à produire un composé de la structure V 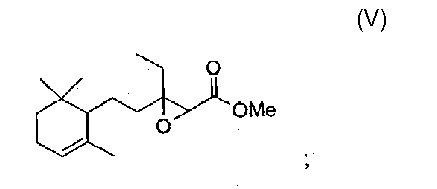
(2) la saponification du composé de la structure V de façon à produire un composé de la structure VI 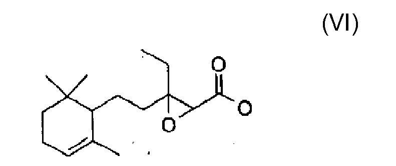
(3) la soumission du composé de la structure VI à une pyrolyse de façon à produire un composé de la structure VII 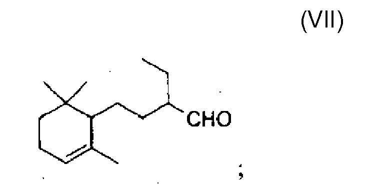
(4) la soumission d'un composé de la structure VII à une réaction de Knoevenagel avec un acide cyanoacétique de façon à produire un composé de la structure VIII 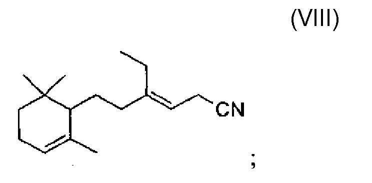
(5) la production d'un composé de la structure IX par la soumission du composé VIII à une réaction de fermeture de cycle 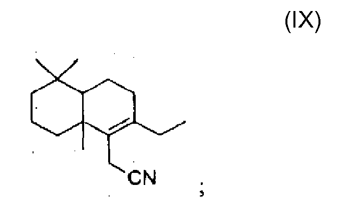
(6) la saponification du composé de la structure IX de façon à produire un composé de la structure X 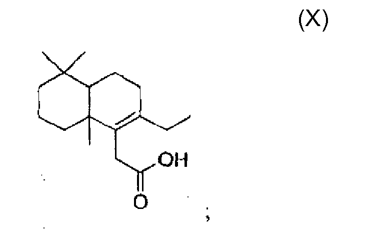
(7) la cyclisation du composé de la structure X de façon à produire un composé de la structure XI 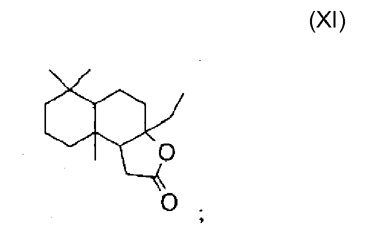 et
(8) la réduction du composé de la structure XI de façon à produire une structure XII 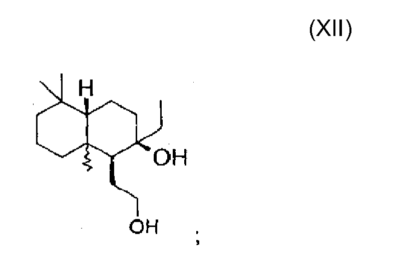 et
(9) la soumission d'un mélange diastéréo-isomérique de la structure XII à une réaction de fermeture de cycle de façon à fournir le mélange diastéréo-isomérique de la structure I :

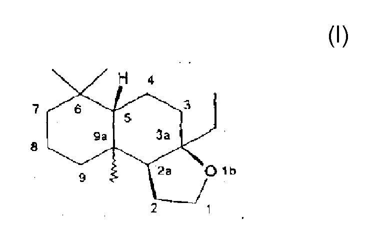

2. Un processus de préparation d'un composant isomérique de la formule 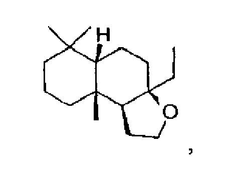 comprenant les opérations suivantes :
(1) la soumission d'un composé de la structure IV 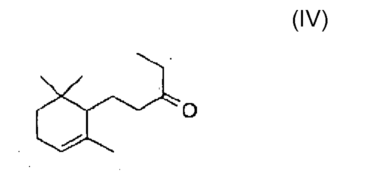 à une condensation de Darzens de façon à produire un composé de la structure V 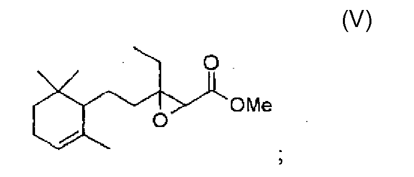
(2) la saponification du composé de la structure V de façon à produire un composé de la structure VI 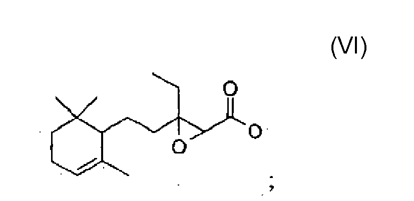
(3) la soumission de la structure VI à une pyrolyse de façon à produire un composé de la structure VII 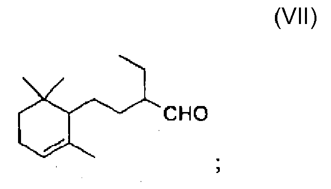
(4) la soumission d'un composé de la structure VII à une réaction de Knoevenagel avec un acide cyanoacétique de façon à produire un composé de la structure VIII 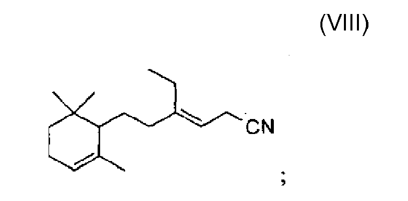
(5) la production d'un composé de la structure IX par la soumission du composé VIII à une réaction de fermeture de cycle 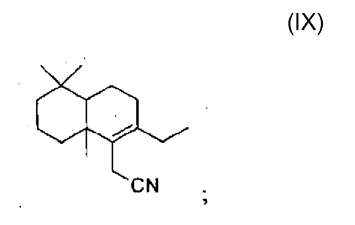
(6) la saponification du composé de la structure IX de façon à produire un composé de la structure X 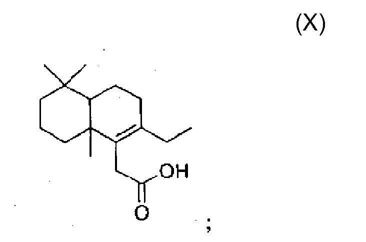
(7) la cyclisation du composé de la structure X de façon à produire un composé de la structure XI 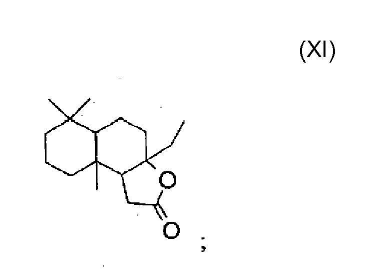 et
(8) la réduction du composé de la structure XI de façon à fournir la structure XII 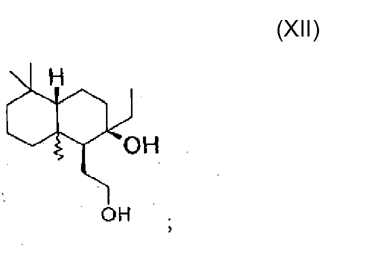 et
(9) la cristallisation du composé de la structure XII de façon à former des cristaux et la soumission des cristaux à une réaction de fermeture de cycle et l'obtention de l'isomère cis de la structure II :

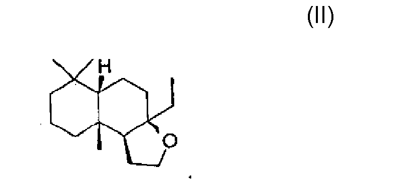

3. Un processus de préparation d'un composant isomérique de la formule 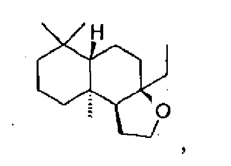 comprenant les opérations suivantes :
(1) la soumission d'un composé de la structure IV 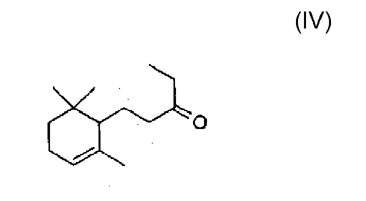 à une condensation de Darzens de façon à produire un composé de la structure V 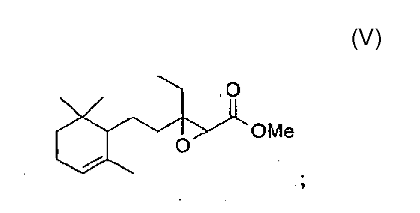
(2) la saponification du composé de la structure V de façon à produire un composé de la structure VI 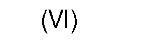 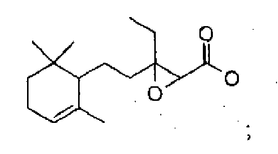
(3) la soumission du composé de la structure VI à une pyrolyse de façon à produire un composé de la structure VII 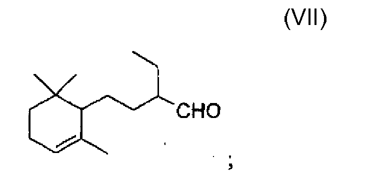
(4) la soumission d'un composé de la structure VII à une réaction de Knoevenagel avec un acide cyanoacétique de façon à produire un composé de la structure VIII 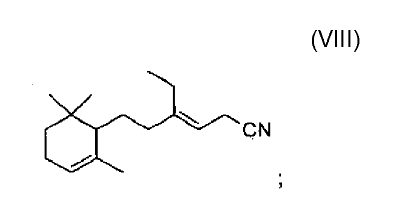
(5) la production d'un composé de la structure IX par la soumission du composé VIII à une réaction de fermeture de cycle 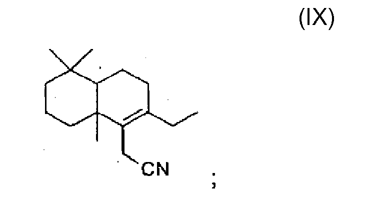
(6) la saponification du composé de la structure IX de façon à produire un composé de la structure X 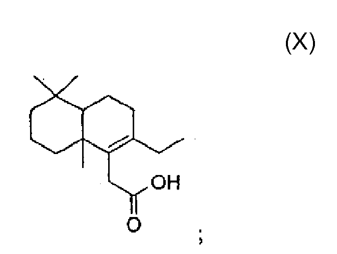
(7) la cyclisation du composé de la structure X de façon à produire un composé de la structure XI 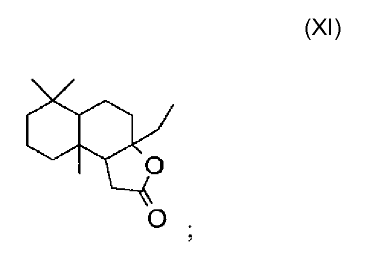 et
(8) la réduction du composé de la structure XI de façon à fournir la structure XII 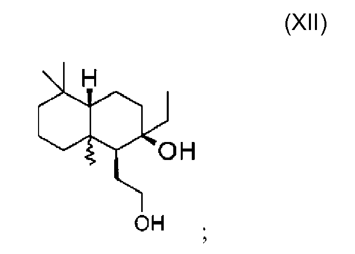
(9) la cristallisation du composé de la structure XII de façon à former des cristaux et la soumission des cristaux à une réaction de fermeture de cycle et l'obtention de l'isomère cis de la structure II : 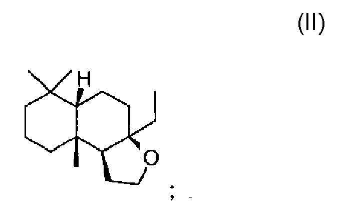 et
(10) la cristallisation de l'échantillon restant contenant la structure XII de façon à former des cristaux et la soumission des cristaux à une réaction de fermeture de cycle et l'obtention de l'isomère trans de la structure III :

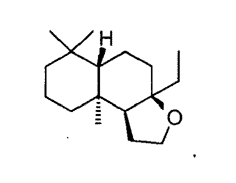

4. Un composé: 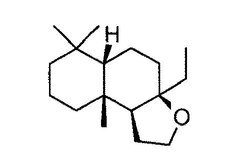 possédant une pureté d'au moins :
(i) 70%, ou
(ii) 80%, ou
(iii) 90%, ou
(iv) 95%, ou
(v) 97,5%, ou
(vi) 99%.

5. Un composé: 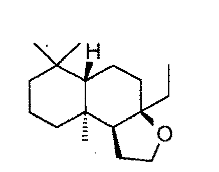 possédant une pureté d'au moins :
(i) 70%, ou
(ii) 80%, ou
(iii) 90%, ou
(iv) 95%, ou
(v) 97,5%, ou
(vi) 99%.

6. Un procédé d'amélioration, d'augmentation ou de modification d'une formulation de fragrance par l'ajout d'une quantité acceptable sur le plan olfactif d'un composé : 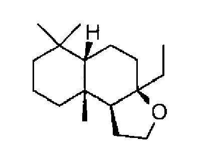 où le composé possède une pureté d'au moins :
(i) 70%, ou
(ii) 80%, ou
(iii) 90%, ou
(iv) 95%, ou
(v) 97,5%, ou
(vi) 99%.

7. Un procédé d'amélioration, d'augmentation ou de modification d'une formulation de fragrance par l'ajout d'une quantité acceptable sur le plan olfactif d'un composé : 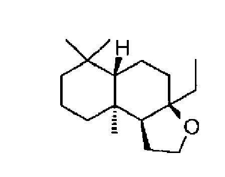 où le composé possède une pureté d'au moins :
(i) 70%, ou
(ii) 80%, ou
(iii) 90%, ou
(iv) 95%, ou
(v) 97,5%, ou
(vi) 99%.

8. Un procédé d'amélioration, d'augmentation ou de modification d'une formulation de fragrance par l'ajout d'une quantité acceptable sur le plan olfactif des composés suivants : 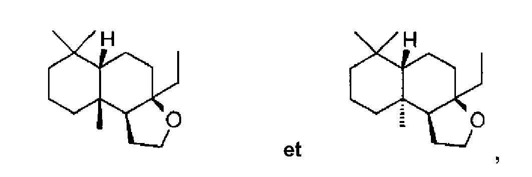 où chaque composé possède une pureté d'au moins :
(i) 70%, ou
(ii) 80%, ou
(iii) 90%, ou
(iv) 95%, ou
(v) 97,5%, ou
(vi) 99%.

9. Un mélange diastéréo-isomère de la structure : 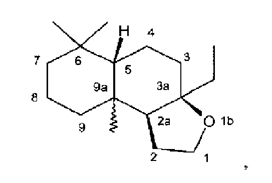 où le mélange diastéréo-isomère possède un rapport isomère cis/trans de :
(i) 30/50 à 50/30, ou
(ii) 35/45 à 45/35, ou
(iii) 41/34, ou
(iv) 30/45.

10. Un procédé d'amélioration, d'augmentation ou de modification d'une formulation de fragrance par l'ajout d'une quantité acceptable sur le plan olfactif d'un mélange diastéréo-isomère de la structure : 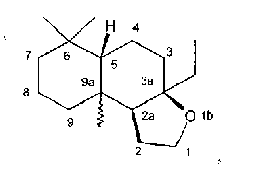 où le mélange diastéréo-isomère possède un rapport isomère cis/trans de :
(i) 30/50 à 50/30, ou
(ii) 35/45 à 45/35, ou
(iii) 41/34, ou
(iv) 30/45.
